Europäisches Patentamt

European Patent Office

Office européen des brevets

(11) Publication number: **0 372 676**
**A1**

## (12) EUROPEAN PATENT APPLICATION

(21) Application number: **89305729.9**

(22) Date of filing: **07.06.89**

(51) Int. Cl.⁵: **A61K 33/30, //(A61K33/30, 31:295,33:00,31:60,31:44,31:18)**

(30) Priority: **08.12.88 AU 1849/88**

(43) Date of publication of application:
**13.06.90 Bulletin 90/24**

(84) Designated Contracting States:
**CH DE ES FR GB GR IT LI NL SE**

(71) Applicant: **Carantinos, Spyros**
**10, Linsley Street**
**Gladesville New South Wales(AU)**

(72) Inventor: **Carantinos, Spyros**
**10, Linsley Street**
**Gladesville New South Wales(AU)**

(74) Representative: **Baillie, Iain Cameron et al**
**c/o Ladas & Parry Isartorplatz 5**
**D-8000 München 2(DE)**

(54) **Therapeutic preparation and method.**

(57) This invention relates to a therapeutic preparation comprising ferric ammonium citrate and zinc oxide and, optionally, aspirin, sodium bicarbonate, citric acid, chlorpropamide and/or Vitamin B1, to a method of preparing it and to a method of treating a diseased mammal, comprising administering to the diseased mammal a pharmaceutically effective amount of the therapeutic preparation.

EP 0 372 676 A1

# THERAPEUTIC PREPARATION AND METHOD

This invention relates to a therapeutic preparation, a method of preparing it and to a method of treating diseased mammals.

This invention provides a therapeutic preparation comprising a mixture of ferric ammonium citrate and zinc oxide. Preferably, the preparation of the invention also includes aspirin (acetylsalicylic acid), sodium bicarbonate and citric acid. It is found that the preparation works more quickly if these components are included. Optionally, chlorpropamide and/or Vitamin B1 may be included for added efficacy.

The aspirin, sodium bicarbonate and citric acid components which are in the preferred form of the therapeutic preparation of the invention are found in convenient proportions in the commercially available preparation "Alka-Seltzer" (trade mark) and this commercial preparation may be used in the preparation of the invention.

The optional chlorpropamide component is also available commercially under the trade marks Diabinese and Promide -prescribed for treatment of uncomplicated diabetes mellitus. Vitamin B1 is commonly available commercially.

The proportions of each component in the preparation of the invention may be varied to suit the subject being treated, as will be discussed below, and as will be readily apparent to one skilled in the art. Example 1, below, sets out the preferred proportions for treatment of humans. These proportions can be expressed as a percentage by weight, as follows:
7 - 8% aspirin,
41 - 42% sodium bicarbonate,
22 - 23% citric acid,
17 - 18% ferric ammonium citrate and
11 - 13% zinc oxide.

This invention also provides a method of preparing a therapeutic preparation, comprising dissolving or suspending in water, or another suitable carrier, ferric ammonium citrate and zinc oxide, and, optionally, aspirin, sodium bicarbonate and citric acid.

In addition, this invention provides a method of treating a diseased mammal, comprising administering to the diseaed mammal a pharmaceutically effective amount of ferric ammonium citrate and zinc oxide, with the optional addition of aspirin, sodium bicarbonate, citric acid, chlorpropamide and/or Vitamin B1.

Tests which have been carried out indicate that the preparation of the invention is capable of treating one or more of the following diseases: arthritis, bronchitis, diabetes, arteriosclerosis, broken bones, Parkinson's disease, high blood cholesterol, cirrhosis of the liver, enlargement of the prostate gland. Often, more than one of these diseases may be treated simultaneously. It is believed that the preparation of the invention may be capable of treating other diseases, perhaps even acquired immuno-deficiency syndrome ("AIDS"), but suitable tests have not yet been carried out. Similarly, the preparation of the invention may also neutralise radiation, even after a nuclear explosion.

It is thought that the preparation of the invention has a beneficial effect on mammalian cells, perhaps changing the ionisation of the atoms in the cells from positive (in which state the cell may be uncontrollable and vulnerable to disease) to negative (when the cell is under control and healthy).

Whether the above theory is accurate or not, it is certainly the case that the effect of the combination of ferric ammonium citrate and zinc oxide is quite different from expectations based on a knowledge of the individual components. It is apparent that the components work together in synergy to produce a quite unexpected result.

The invention will now be described in connection with examples thereof, which are not to be regarded as limiting on the scope of the invention.

EXAMPLE 1

The following were dissolved or suspended in 100 ml water:
324 mg aspirin, 1900 mg sodium bicarbonate and 1050 mg citric acid (obtained as 1 tablet of "Alka-Seltzer" - trade mark);
825 mg ferric ammonium citrate (B.P.); and
515 mg zinc oxide (B.P.).
The resultant solution/suspension was suitable for ingestion as a draught.

EXAMPLE 2

In this Example, the preparation comprised 1 tablet of "Alka-Seltzer" (trade mark), containing 324 mg aspirin, 1900 mg sodium bicarbonate and 1050 mg citric acid; 825 mg ferric ammonium citrate (B.P.); 515 mg zinc oxide (B.P.), one tablet of chlorpropamide (250 mg) and two tablets of vitamin B1.

EXAMPLE 3

The preparation of Example 2 was varied by providing an extra two tablets of aspirin of approximately 300 mg each. This combination is useful for cases of severe diabetes. It is also useful to increase the amount of zinc oxide in such cases.

## EXAMPLE 4

Tests have shown that the preparations described in Examples 1 to 3 may be administered once a day, or, in half the given quantities, twice a day. For children under 12 years of age, the daily dose should be half that of adults. For other mammals, the dose should be adjusted for body weight; experimentation will show the correct dose, as illustrated by Example 7, below.

## EXAMPLE 5

The test subject was a male caucasion aged 63 years at the commencement of the test. The subject had suffered from malaria for many years and had cirrhosis of the liver. About ten years before commencement of testing the subject had had a "bypass" operation to alleviate arteriosclerosis. The subject lost mobility and developed severe arthritis and gastritis as well as colon cancer. During surgical treatment for colon cancer, it was found that the subject was diabetic. The subject also suffered from bronchitis every winter and commenced to develop an enlarged prostate.

The subject took small amounts of ferric ammonium citrate and zinc oxide each day, as well as 2 tablets of "Alka-Seltzer" two or three times a day, Vitamin B1 was taken 3 times a day and 1 tablet of aspirin daily. After 21 days, the diabetes disappeared.

The subject continued to take the above combination, but the quantity of ferric ammonium citrate and zinc oxide was gradually increased to 825 mg and 515 mg, respectively, per day ("the optimum dose"). The subject noticed an increase in energy.

Zinc oxide was then omitted from the daily dose and copper oxide or sulphate was substituted. The diabetes returned. The diabetes disappeared again once the zinc oxide component was restored to the dose.

The subject continued to take the optimum dose and over time the arthritis symptoms were substantially alleviated. After the subject had beeen taking the dose for almost five years, analysis showed low blood cholesterol, no arteriosclerosis and no cirrhosis of the liver. In addition, the subject no longer suffered from bronchitis and was not susceptible to influenza. The subject's prostate problems disappeared after a programme of pelvic floor exercises. The subject enjoys a feeling of general well-being and vigour which is quite unusual, given the subject's age and medical background.

## EXAMPLE 6

The subject was a female caucasian of 70 years at the commencement of treatment. The subject had suffered a slight stroke shortly before and had Parkinson's disease, with the associated trembling symptoms.

After taking the preparation of Example 1 as well as Vitamin B1 once daily, there was a slow but definite improvement in the subject, so that after six months the subject's tremors were reduced by 50% and after one year, the symptoms were reduced by 80 to 90%.

## EXAMPLE 7

A pair of fillies, one with a broken pelvis and the other with a broken hip, were dosed daily with a preparation comprising zinc oxide, ferric ammonium citrate and vitamins. In addition, two spoons of sulphur were given to the fillies once a week. At first, relatively small amounts of zinc oxide and ferric ammonium citrate were administered, then the dose was increased daily until it reached a maximum of 5.15 g zinc oxide and 8.25 g ferric ammonium citrate, per day, i.e., ten times the quantity of these components in the preparation of Example 1. The treatment was successful; the fillies improved, the breaks healed and both fillies became energetic and could gallop actively.

It will be apparent to one skilled in the art that the proportions of the components of the therapeutic preparation of this invention may be variable and are not limited to the precise amounts or percentages indicated herein. Moreover, the method of administration of the therapeutic preparation may be variable; future tests may show that the preparation may be administered in suppository form, for example.

In addition, it is contemplated that the preparation of the invention may well be effective against diseases not referred to herein.

## Claims

1. A therapeutic preparation comprising ferric ammonium citrate and zinc oxide and, optionally, aspirin, sodium bicarbonate and citric acid.

2. A therapeutic preparation as claimed in claim 1 wherein the preparation contains 7 - 8% aspirin, 41 - 42% sodium bicarbonate, 22 - 23% citric acid, 17 - 18% ferric ammonium citrate and 11 - 13% zinc oxide, by weight.

3. A therapeutic preparation as claimed in claim 1 or 2, wherein the preparation also includes ohlorpropamide.

4. A therapeutic preparation as claimed in any one of claims 1 to 3, wherein the preparation also includes vitamin B1.

5. A method of preparing a therapeutic preparation, comprising dissolving or suspending in water, or another suitable carrier, ferric ammonium citrate and zinc oxide and, optionally, aspirin, sodium bicarbonate and citric acid.

6. A method as claimed in claim 5, wherein 7 - 8% aspirin, 41 - 42% sodium bicarbonate, 22 - 23% citric acid, 17 - 18% ferric ammonium citrate and 11 - 13% zinc oxide, by weight, are dissolved or suspended in 100 ml water.

7. Use of a citrate composition in the preparation of a medicament for treating a diseased mammal, wherein the composition contains a pharmaceutically effective amount of ferric ammonium citrate and zinc oxide, with the optional addition of aspirin, sodium bicarbonate, citric acid, chlorpropamide and/or Vitamin B1.

8. Use as claimed in claim 7, wherein 7 - 8% of aspirin, 41 - 42% sodium bicarbonate, 22 - 23% citric acid, 17 - 18% ferric ammonium citrate and 11 - 13% zinc oxide, by weight, are administered to the diseased mammal.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 225 614  (AKTIEBOLAGET DRACO)<br>* Column 4, example 1 *<br>--- | 1 | A 61 K  33/30 //<br>(A 61 K  33/30<br>A 61 K  31:295<br>A 61 K  33:00<br>A 61 K  31:60<br>A 61 K  31:44<br>A 61 K  31:18 ) |
| A | EP-A-0 208 362  (THE PROCTER & GAMBLE Co.)<br>* Page 1, lines 1-8; claims 2,4 *<br>----- | 1,3 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 30-01-1990 | BERTOCCHI C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P0401)